# EUROPEAN PATENT APPLICATION

(11) **EP 4 085 908 A1**
(43) Date of publication of application: **09.11.2022**
(21) Application number: 20909191.7
(22) Date of filing: 31.12.2020
(51) Int. Cl.: A61K 31/343, C07D 307/81, A61K 39/395, A61P 35/00

(54) **PHARMACEUTICAL COMBINATION FOR TREATING TUMORS AND APPLICATION THEREOF**

(30) Priority: 31.12.2019 CN 201911417368
(71) Applicant: Fukang (Shanghai) Health Technology Co., Ltd., Shanghai 201318 (CN)
(72) Inventor: SHEN, Xiaokun, Shanghai 201318 (CN); LI, Zeng, Shanghai 201318 (CN)
(74) Representative: Potter Clarkson
(86) International application number: PCT/CN2020/142257
(87) International publication number: WO 2021/136523

(57) **Abstract**

Provided are a pharmaceutical combination for treating tumors and an application thereof. The pharmaceutical combination comprises a compound represented by formula A, a pharmaceutically acceptable salt thereof, a solvate thereof or a solvate of a pharmaceutically acceptable salt thereof, and a "PD-1 inhibitor and/or PD-L1 inhibitor". The components of the pharmaceutical combination, when used in combination, can significantly increase the inhibition rate of each individual drug on tumor growth, and there were no acute adverse reactions in mice after administration, demonstrating that such a combination therapy has good safety and effectiveness.

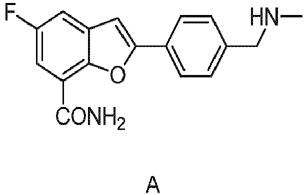

## Description

The present application claims priority to Chinese Patent Application No. 201911417368.7 filed on Dec 31, 2019, which is incorporated herein by reference in its entirety.

### TECHNICAL FIELD

The present invention relates to a pharmaceutical combination for treating a tumor and use thereof.

### BACKGROUND

Precise medicine is an inevitable trend for therapies of malignant tumors and the orientation for future development. Poly (ADP-ribose) polymerase (PARP) is a target of interest for precise tumor medicine concept in recent years. An inhibitor of the target can generate synergistic lethal effect by inhibiting DNA base excision repair (BER) pathway key enzyme PARP1/2 and DNA homologous recombination deficiency (HRD) including BRCA1/2, PTEN, EWS-FLI and the like. Since HRD only occurs in tumor cells and HR in normal cells functions well, PARP inhibitors are highly selective for tumors with HR deficiency. In 2014, PARP inhibitor olaparib was approved in the EU and the United States for treating advanced ovarian cancer, indicating that the feasibility of PARP inhibitors as anti-tumor targets and the theory of synthetic lethality was clinically confirmed for the first time. Currently, there are 4 PARP inhibitors approved worldwide, and olaparib was approved for marketing in China in 2018. However, no original PARP inhibitors have been approved in China. Only a few PARP inhibitors are in early clinical stages.

Patent No. WO2013117120 disclosed a compound, mefuparib hydrochloride (the structural formula of which is 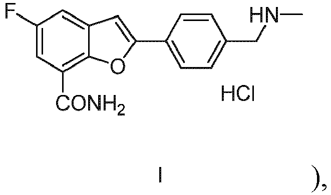 having a novel structure. The parent structure 2-arylfuran is widely present in active natural compounds, and is also one of the chemical dominant structures for drugs, having prospective druggability. The PARP inhibitor has advantages of good water solubility (> 35 mg/mL which is 350 times higher than that of olaparib), simple synthesis and formulation processes, good stability, excellent pharmacokinetics, high tissue distribution, easy penetration through blood brain barrier and the like. These characteristics provide an important basis for mefuparib hydrochloride for treating various malignant tumors.

Tumor immunotherapy enhances the immunity against tumors by regulating the immune system, thereby inhibiting and killing tumor cells. Tumor immunotherapy is one of the most promising orientations in the tumor therapy field. PD-1 (programmed death-1) is found in apoptotic T cell hybridomas and is named as programmed death PD-1 receptor due to its association with apoptosis. PD-1 programmed death receptor is an important immunosuppressive molecule and is a member of the CD28 superfamily. PD-1 is expressed predominantly in activated T cells and B cells, and is a surface receptor on activated T cells. PD-1 has two ligands, PD-L1 (B7-H1) and PD-L2 (B7-DC). Tumor microenvironments in the bodies can induce infiltrated T cells to overexpress PD-1 molecules, and tumor cells can overexpress ligands PD-L1 and PD-L2 of PD-1, leading to continuously activated PD-1 pathway in the tumor microenvironment. When PD-L1 binds to PD-1, the function of T cells is inhibited, and signals for attacking tumors cannot be sent to the immune system. PD-1 inhibitors, including PD-1 antibodies and PD-L1 antibodies, are new tumor immunotherapies. Unlike surgery, chemoradiotherapy and targeted therapies, PD-1 inhibitors do not directly kill cancer cells, but rather act against cancer by activating the patient's own immune system.

Until now, 5 PD-1 inhibitors have been approved in several dozens of countries, including 2 PD-1 antibodies and 3 PD-L1 antibodies, i.e., Opdivo (nivolumab), Keytruda (pembrolizumab), Tecentriq (atezolizumab), Imfinzi (durvalumab) and Bavencio (avelumab). Among these, Opdivo and Keytruda have been approved for marketing in China. In addition, 3 PD-1 inhibitors from enterprises in China have been approved, i.e., toripalimab (Tuoyi) of Junshi, sintilimab (TYVYT) of Innovent, and camrelizumab (Airuika) of Hengrui.

On the San Antonio breast Cancer Symposium held in 2017, the investigator published a striking achievement: the MEDIOLA phase II trial, a clinical trial evaluating a PD-L1 antibody Imfinzi (durvalumab) in combination with a PARP inhibitor olaparib (Lynparza) for treating patients with BRCA-mutant and HER2-negative advanced breast cancer. The data showed that the disease control rate at week 12 was 80%, the response rate at week 28 was 63%, the median DoR at week 28 was 9.2 months, the median PFS at week 28 was 8.2 months, and the disease control rate at week 28 was 50%. The data were superior to those of the patients receiving olaparib monotherapy, in which the response rate was 59.9% and the median PFS was 7.0 months. The most common grade 3 adverse events associated with olaparib were anemia (12%) and neutropenia (6%); the most common grade 3 adverse event associated with durvalumab was pancreatitis (6%).

In the Society of Gynecological Oncology (SGO) 2018 annual meeting, the results of TOPACIO study (PARP inhibitor niraparib in combination with PD-1 inhibitor Keytruda) were reported, showing that PARP inhibitors in combination with PD-1 inhibitors have long-lasting efficacy in patients with ovarian cancer and triple-negative breast cancer resistant/refractory to platinum-based drugs. In ovarian cancer or triple-negative breast cancer patients with secondary or primary resistance to platinum-based drugs, the total ORR (including CR and PR) was 25%, and the DCR (CR + PR + SD) was 68%; in patients with primary resistance to platinum-based drugs, the ORR was 24%. The efficacy of the patient is independent of the status of the markers: in BRCA wild-type patients, the ORR was 26% (9/34); in HRD-negative patients, the ORR was 29% (7/24). Currently, the data are insufficient for the duration of response. 60% responding patients (9/15) are still receiving the treatment and more than half of the patients with controlled disease have been treated for more than 6 months. For side effects, 32 in the 58 subjects had grade 3 or higher adverse events (55%), with anemia (18%), thrombocytopenia (15%) and fatigue (4%) being the most common grade 3 or higher adverse events. The adverse events mainly came from the PARP inhibitor. In addition, 15% (8) patients had immune-related adverse events.

In summary, use of PARP inhibitors in combination with PD-1 antibodies in treating cancers has significant therapeutic benefit in the clinic while still presenting a greater risk of side effects, which are mainly derived from PARP inhibitors according to the existing clinical data. Therefore, combinations of PARP inhibitors and PD-1 antibodies have a broad marketing prospect for the treatment of cancer, while better clinical efficacy and lower side effects are still the inevitable trends of clinical development.

### SUMMARY

The present invention provides a pharmaceutical combination for treating a tumor and used thereof, which are different from the prior art. The combined use of the components of the pharmaceutical combination can provide a significantly superior tumor growth inhibition relative to the monotherapies. The treated mice showed no acute adverse events, suggesting that the combination therapy has good safety and efficacy.

The present invention provides a pharmaceutical combination, comprising: a compound of formula A, a pharmaceutically acceptable salt thereof, a solvate thereof or a solvate of the pharmaceutically acceptable salt thereof; and "a PD-1 inhibitor and/or a PD-L1 inhibitor";

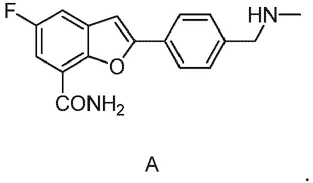

In one embodiment, the pharmaceutically acceptable salt of the compound of formula A is mefuparib hydrochloride.

In one embodiment, the PD-1 inhibitor may be one or more of a PD-1 antibody, a PD-1 polypeptide inhibitor and a PD-1 micromolecular inhibitor, preferably one or more of toripalimab, sintilimab, camrelizumab, pembrolizumab and nivolumab, and more preferably toripalimab.

In one embodiment, the PD-L1 inhibitor may be one or more of a PD-L1 antibody, a PD-L1 polypeptide inhibitor and a PD-L1 micromolecular inhibitor, and preferably one or more of atezolizumab (trade name Tecentriq), durvalumab (trade name Imfinzi) and alirocumab (trade name Bavencio).

In one embodiment, "the PD-1 inhibitor and/or the PD-L1 inhibitor" and the compound of formula A, the pharmaceutically acceptable salt thereof, the solvate thereof or the solvate of the pharmaceutically acceptable salt thereof may be administered simultaneously or separately. The separate administration may also be a sequential administration.

The simultaneous administration may be a simultaneous administration by containing "the PD-1 inhibitor and/or the PD-L1 inhibitor" and "the compound of formula A, the pharmaceutically acceptable salt thereof, the solvate thereof or the solvate of the pharmaceutically acceptable salt thereof" in a sole pharmaceutical combination; alternatively, a separate pharmaceutical composition containing "the PD-1 inhibitor and/or the PD-L1 inhibitor" and a separate pharmaceutical composition containing "the compound of formula A, the pharmaceutically acceptable salt thereof, the solvate thereof or the solvate of the pharmaceutically acceptable salt thereof" are administered simultaneously.

The separate administration may be separated administrations of a separate pharmaceutical composition containing "the PD-1 inhibitor and/or the PD-L1 inhibitor" and a separate pharmaceutical composition containing "the compound of formula A, the pharmaceutically acceptable salt thereof, the solvate thereof or the solvate of the pharmaceutically acceptable salt thereof" at different time. Separated administrations may be close in time or remote in time.

The sequential administration may be administration of one of a separate pharmaceutical composition containing "the PD-1 inhibitor and/or the PD-L1 inhibitor" and a separate pharmaceutical composition containing "the compound of formula A, the pharmaceutically acceptable salt thereof, the solvate thereof or the solvate of the pharmaceutically acceptable salt thereof" and subsequent administration of the other. Separated administrations may be close in time or remote in time.

The administration regimens (including route of administration, dose, interval, etc.) of "the PD-1 inhibitor and/or the PD-L1 inhibitor" and "the compound of formula A, the pharmaceutically acceptable salt thereof, the solvate thereof or the solvate of the pharmaceutically acceptable salt thereof', whether administered simultaneously or separately, may be identical or different, and may be adjusted as necessary by those skilled in the art to provide the best therapeutic effect.

In one embodiment, the pharmaceutical combination comprises mefuparib hydrochloride and toripalimab.

The present invention further provides a pharmaceutical composition X comprising:
the compound of formula A, the pharmaceutically acceptable salt thereof, the solvate thereof or the solvate of the pharmaceutically acceptable salt thereof;
"the PD-1 inhibitor and/or the PD-L1 inhibitor";
and a pharmaceutically acceptable excipient.

The pharmaceutical composition can be prepared into various suitable dosage forms according to different administration modes, including enteral administration dosage forms (such as oral dosage forms (tablets, pills, capsules, powder and granules) and gaseous dosage forms (inhalants)) and parenteral administration dosage forms (such as injections, pastes and emulsions).

In one embodiment, the pharmaceutical composition X is in an injectable dosage form or an oral dosage form. The oral dosage form may be a tablet dosage form.

The present invention further provides a pharmaceutical composition Y comprising a first pharmaceutical composition and a second pharmaceutical composition; wherein
the first pharmaceutical composition comprises: the compound of formula A, the pharmaceutically acceptable salt thereof, the solvate thereof or the solvate of the pharmaceutically acceptable salt thereof described above, and a pharmaceutically acceptable excipient;
the second pharmaceutical composition comprises: "the PD-1 inhibitor and/or the PD-L1 inhibitor" described above, and a pharmaceutically acceptable excipient.

In one embodiment, the first pharmaceutical composition is in an oral dosage form, preferably in a tablet dosage form.

In one embodiment, the second pharmaceutical composition is in an injectable dosage form.

In one embodiment, the first pharmaceutical composition is in a tablet dosage form, and the second pharmaceutical composition is in an injectable dosage form. The present invention further provides a kit, comprising:
a first container comprising the first pharmaceutical composition described above; and
a second container comprising the second pharmaceutical composition described above.

The present invention further provides use of a substance M in preparing a medicament for preventing and/or treating a tumor, wherein the substance M is the pharmaceutical combination described above, the pharmaceutical composition X described above, or the pharmaceutical composition Y described above.

The tumor may be a solid tumor and/or a hematological tumor. The solid tumor may be one or more of lung cancer, colon cancer, rectal cancer, breast cancer, prostate cancer, liver cancer, pancreatic cancer, brain cancer, kidney cancer, ovarian cancer, stomach cancer, skin cancer, bone cancer, neuroglioma, glioblastoma, hepatocellular carcinoma, papillary kidney cancer, head and neck cancer, leukemia, lymphoma, myeloma and multiple myeloma.

In the use, the administration regimens (including route of administration, dose, interval, etc.) of "the compound of formula A, the pharmaceutically acceptable salt thereof, the solvate thereof or the solvate of the pharmaceutically acceptable salt thereof" and "the PD-1 inhibitor and/or the PD-L1 inhibitor" may be identical or different, and may be adjusted as necessary by those skilled in the art to provide the best therapeutic effect.

The dose of "the compound of formula A, the pharmaceutically acceptable salt thereof, the solvate thereof or the solvate of the pharmaceutically acceptable salt thereof" and "the PD-1 inhibitor and/or the PD-L1 inhibitor" can be determined according to the body weight of the subject.

In one embodiment, "the compound of formula A, the pharmaceutically acceptable salt thereof, the solvate thereof or the solvate of the pharmaceutically acceptable salt thereof" and "the PD-1 inhibitor and/or the PD-L1 inhibitor" are administered simultaneously or separately.

In one embodiment, "the compound of formula A, the pharmaceutically acceptable salt thereof, the solvate thereof or the solvate of the pharmaceutically acceptable salt thereof" is administered at a dose of 100-1000 mg.

In one embodiment, the compound of formula A, the pharmaceutically acceptable salt thereof, the solvate thereof or the solvate of the pharmaceutically acceptable salt thereof is administered at a frequency of 0.5-2 doses/day.

In one embodiment, the compound of formula A, the pharmaceutically acceptable salt thereof, the solvate thereof or the solvate of the pharmaceutically acceptable salt thereof is administered orally.

In one embodiment, "the PD-1 inhibitor and/or the PD-L1 inhibitor" is administered at a dose of 50-500 mg.

In one embodiment, "the PD-1 inhibitor and/or the PD-L1 inhibitor" is administered at a frequency of once every 7-31 days.

In one embodiment, "the PD-1 inhibitor and/or the PD-L1 inhibitor" is administered orally or by injection.

In one embodiment, the compound of formula A, the pharmaceutically acceptable salt thereof, the solvate thereof or the solvate of the pharmaceutically acceptable salt thereof is administered orally at a dose of 100-1000 mg and at a frequency of 0.5-2 doses/day;
and "the PD-1 inhibitor and/or the PD-L1 inhibitor" is administered by injection at a dose of 50-500 mg and at a frequency of once every 7-31 days.

The present invention further provides use of the compound of formula A, the pharmaceutically acceptable salt thereof, the solvate thereof or the solvate of the pharmaceutically acceptable salt thereof described above in preparing a medicament for treating a tumor, wherein "the compound of formula A, the pharmaceutically acceptable salt thereof, the solvate thereof or the solvate of the pharmaceutically acceptable salt thereof" is used in combination with "the PD-1 inhibitor and/or the PD-L1 inhibitor";

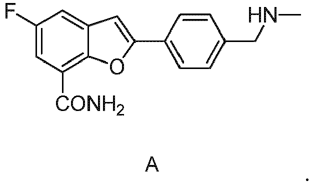

"The compound of formula A, the pharmaceutically acceptable salt thereof, the solvate thereof or the solvate of the pharmaceutically acceptable salt thereof" and "the PD-1 inhibitor and/or the PD-L1 inhibitor" may be administered simultaneously or separately. The separate administration may also be a sequential administration.

The compound of formula A, the pharmaceutically acceptable salt thereof, the solvate thereof or the solvate of the pharmaceutically acceptable salt thereof may be administered orally.

The compound of formula A, the pharmaceutically acceptable salt thereof, the solvate thereof or the solvate of the pharmaceutically acceptable salt thereof may be administered at a dose of 100-1000 mg.

The compound of formula A, the pharmaceutically acceptable salt thereof, the solvate thereof or the solvate of the pharmaceutically acceptable salt thereof may be administered at a frequency of 0.5-2 doses/day.

"The PD-1 inhibitor and/or the PD-L1 inhibitor" may be administered orally or by injection.

"The PD-1 inhibitor and/or the PD-L1 inhibitor" may be administered at a dose of 50-500 mg.

"The PD-1 inhibitor and/or the PD-L1 inhibitor" may be administered at a frequency of once every 7-31 days.

The tumor may be a solid tumor and/or a hematological tumor. The solid tumor may be one or more of lung cancer, colon cancer, rectal cancer, breast cancer, prostate cancer, liver cancer, pancreatic cancer, brain cancer, kidney cancer, ovarian cancer, stomach cancer, skin cancer, bone cancer, neuroglioma, glioblastoma, hepatocellular carcinoma, papillary kidney cancer, head and neck cancer, leukemia, lymphoma, myeloma and multiple myeloma.

In one embodiment, the compound of formula A, the pharmaceutically acceptable salt thereof, the solvate thereof or the solvate of the pharmaceutically acceptable salt thereof is administered orally at a dose of 100-1000 mg and at a frequency of 0.5-2 doses/day;
and "the PD-1 inhibitor and/or the PD-L1 inhibitor" is administered by injection at a dose of 50-500 mg and at a frequency of once every 7-31 days.

The present invention further provides use of "the PD-1 inhibitor and/or the PD-L1 inhibitor" in preparing a medicament for treating a tumor, wherein "the PD-1 inhibitor and/or the PD-L1 inhibitor" is used in combination with "the compound of formula A, the pharmaceutically acceptable salt thereof, the solvate thereof or the solvate of the pharmaceutically acceptable salt thereof";

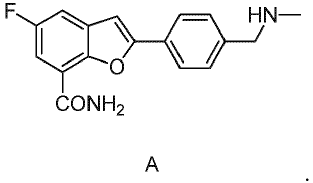

"The compound of formula A, the pharmaceutically acceptable salt thereof, the solvate thereof or the solvate of the pharmaceutically acceptable salt thereof" and "the PD-1 inhibitor and/or the PD-L1 inhibitor" may be administered simultaneously or separately. The separate administration may also be a sequential administration.

"The PD-1 inhibitor and/or the PD-L1 inhibitor" may be administered orally or by injection.

"The PD-1 inhibitor and/or the PD-L1 inhibitor" may be administered at a dose of 50-500 mg.

"The PD-1 inhibitor and/or the PD-L1 inhibitor" may be administered at a frequency of once every 7-31 days.

The compound of formula A, the pharmaceutically acceptable salt thereof, the solvate thereof or the solvate of the pharmaceutically acceptable salt thereof may be administered orally.

The compound of formula A may be administered at a dose of 100-1000 mg.

The compound of formula A may be administered at a frequency of 0.5-2 doses/day.

The tumor may be a solid tumor and/or a hematological tumor. The solid tumor may be one or more of lung cancer, colon cancer, rectal cancer, breast cancer, prostate cancer, liver cancer, pancreatic cancer, brain cancer, kidney cancer, ovarian cancer, stomach cancer, skin cancer, bone cancer, neuroglioma, glioblastoma, hepatocellular carcinoma, papillary kidney cancer, head and neck cancer, leukemia, lymphoma, myeloma and multiple myeloma.

In one embodiment, the compound of formula A is administered orally at a dose of 100-1000 mg and at a frequency of 0.5-2 doses/day;
and "the PD-1 inhibitor and/or the PD-L1 inhibitor" is administered by injection at a dose of 50-500 mg and at a frequency of once every 7-31 days.

The present invention further provides a method for treating or preventing a tumor, comprising administering to a subject an effective amount of a substance M, wherein the substance M is the pharmaceutical combination described above, the pharmaceutical composition X described above, or the pharmaceutical composition Y described above.

In the method, the tumor may be a solid tumor and/or a hematological tumor. The solid tumor may be one or more of lung cancer, colon cancer, rectal cancer, breast cancer, prostate cancer, liver cancer, pancreatic cancer, brain cancer, kidney cancer, ovarian cancer, stomach cancer, skin cancer, bone cancer, neuroglioma, glioblastoma, hepatocellular carcinoma, papillary kidney cancer, head and neck cancer, leukemia, lymphoma, myeloma and multiple myeloma.

In the method, "the compound of formula A, the pharmaceutically acceptable salt thereof, the solvate thereof or the solvate of the pharmaceutically acceptable salt thereof" and "the PD-1 inhibitor and/or the PD-L1 inhibitor" are administered through the same regimen (including route of administration, dose, frequency, etc.) as described above.

The term "PD-1 antibody" refers to an antibody that can bind to PD-1 on the surface of a T cell and blocks the binding of PD-1 to its ligand. PD-1 is known as programmed cell death protein 1 and is an important immunosuppressive molecule.

The term "PD-1 polypeptide inhibitor" refers to a polypeptide that can bind to PD-1 on the surface of a T cell and blocks the binding of PD-1 to its ligand.

The term "PD-1 micromolecular inhibitor" refers to a chemical micromolecule that can bind to PD-1 on the surface of a T cell and blocks the binding of PD-1 to its ligand.

The term "PD-L1 antibody" refers to an antibody that can bind to PD-L1 on the surface of a tumor cell and blocks the binding of PD-L1 to PD-1 on T cell surface. PD-L1 is referred to as programmed cell death protein 1 ligand 1.

The term "PD-L1 polypeptide inhibitor" refers to a polypeptide that can bind to PD-L1 on the surface of a tumor cell and blocks the binding of PD-L1 to PD-1 on T cell surface.

The term "PD-L1 micromolecular inhibitor" refers to a micromolecular compound that can bind to PD-L1 on the surface of a tumor cell and blocks the binding of PD-L1 to PD-1 on T cell surface. "Micromolecular compound" refers to a compound having a molecular weight less than 1000.

The term "pharmaceutically acceptable salt" refers to a salt prepared with the compound and a relatively non-toxic and pharmaceutically acceptable acid or base. When the compound contains a relatively acidic functional group, a base addition salt can be obtained by contacting a neutral form of the compound with a sufficient amount of pharmaceutically acceptable base in a pure solution or a suitable inert solvent. Pharmaceutically acceptable base addition salts include, but are not limited to: lithium salt, sodium salt, potassium salt, calcium salt, aluminum salt, magnesium salt, zinc salt, bismuth salt, ammonium salt, and diethanolamine salt. When the compound contains a relatively basic functional group, an acid addition salt can be obtained by contacting a neutral form of the compound with a sufficient amount of pharmaceutically acceptable acid in a pure solution or a suitable inert solvent. The pharmaceutically acceptable acid includes inorganic acids including, but not limited to: hydrochloric acid, hydrobromic acid, hydroiodic acid, nitric acid, carbonic acid, bicarbonate, phosphoric acid, monohydrogen phosphate, dihydrogen phosphate, phosphorous acid, sulfuric acid, hydrogen sulfate, and the like. The pharmaceutically acceptable acid includes organic acids including, but not limited to: acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, salicylic acid, tartaric acid, methanesulfonic acid, isonicotinic acid, acidic citric acid, oleic acid, tannic acid, pantothenic acid, bitartrate, ascorbic acid, gentisic acid, fumaric acid, gluconic acid, saccharic acid, formic acid, ethanesulfonic acid, pamoic acid (i.e., 4,4'-methylene-bis(3-hydroxy-2-naphthoic acid)), amino acids (e.g., glutamic acid, arginine), and the like. When a compound contains both relatively acidic functional group and relatively basic functional group, it can be converted to either a base addition salt or an acid addition salt. Details can be found in Berge et al., "Pharmaceutical Salts", Journal of Pharmaceutical Science 66:1-19 (1977), or, Handbook of Pharmaceutical Salts: Properties, Selection, and Use (P. Heinrich Stahl and Camile G. Wermuth, ed., Wiley-VCH, 2002).

The term "solvate" refers to a substance formed upon crystallization of a compound with a solvent (including, but not limited to: water, methanol, ethanol, etc.). The solvate include stoichiometric and non-stoichiometric solvates.

The term "solvate of the pharmaceutically acceptable salt" refers to a substance formed by combining the compound with a pharmaceutically acceptable (relatively non-toxic, safe, suitable for use in a patient) acid or base in a solvent (including, but not limited to: water, methanol, ethanol, etc.), wherein the pharmaceutically acceptable salt is as defined by the "pharmaceutically acceptable salt" above and the solvent is stoichiometric or non-stoichiometric. The solvate of the pharmaceutically acceptable salt includes, but is not limited to, hydrochloride monohydrate.

The term "therapeutically effective amount" refers to an amount of the compound administered to a patient and sufficient to effectively treat a disease. The therapeutically effective amount will vary depending on the compound, the type of disease, the severity of the disease, the age of the patient, and the like, but can be adjusted as appropriate by those skilled in the art.

The above preferred conditions may be combined arbitrarily to obtain preferred embodiments of the present invention without departing from the general knowledge in the art.

The reagents and starting materials used in the present invention are commercially available.

The positive and progressive effects of the present invention are as follows: the pharmaceutical composition of the present invention demonstrates a tumor growth inhibition up to 60% and causes no acute adverse effects in subjects during the treatment period.

### BRIEF DESCRIPTION OF THE DRAWINGS

FIG. 1 shows the percentage change in body weight of the experimental animals after start of treatment.
FIG. 2 shows the tumor growth curve in the experimental animals after start of treatment.
FIG. 3 shows the tumor volume in the experimental animals after treatment (PG-D24), where "-" indicates the median.
FIG. 4 shows the FACS assay results of hCD45 in peripheral blood of the experimental animals 3 weeks after PBMC inoculation, where "-" indicates the median.

### DETAILED DESCRIPTION

The present invention is further illustrated by the following examples, which are not intended to limit the present invention. Experimental procedures without specified conditions in the following examples were performed in accordance with conventional procedures and conditions, or in accordance with instructions.

### 1. Test compounds

Test compound 1: Tuoyi (toripalimab, PD-1 monoclonal antibody) injection.
Test compound 2: mefuparib hydrochloride.

### 2. Preparation of test compounds

### 2.1 Solvent selection: Tuoyi PD-1 monoclonal antibody injection: PBS; mefuparib hydrochloride: normal saline.

### 2.2 Preparation:

**Table 1. Preparation and storage of test compounds**

| Test compound | Preparation | Final concentration (mg/mL) | Storage |
|---|---|---|---|
| Tuoyi PD-1 monoclonal antibody injection | A proper volume of sample was measured and added into a glass flask by using a microsyringe. A proper volume of PBS was added into the glass flask, and the mixture was well mixed. | 1 | Prepared before use |
| Mefuparib hydrochloride | A proper amount of sample was measured using an electronic balance and added into a glass flask. A proper volume of normal saline was added into the glass flask, and the mixture was well mixed. | 4 | Once a week |

### 3. Materials and instruments

**Table 2. Reagents**

| Name | Unit size | Manufacturer |
|---|---|---|
| Fetal bovine serum (FBS) | 500 mL/bottle | Gibco |
| Leibovitz's L-15 medium | 500 mL/bottle | Gibco |
| Penicillin-streptomycin mixed solution | 100 mL/bottle | Solarbio |
| Trypsin-EDTA digestive juice (0.25%) | 100 mL/bottle | Solarbio |
| Matrigel | 10 mL/bottle | Corning |
| PBS (phosphate-buffered saline) | 500 mL/bottle | Gibco |
| Normal saline (0.9% sodium chloride injection) | 500 mL/bottle | Shij iazhuang No. 4 Pharmaceutical Co., Ltd. |
| PE-Cy7 anti-Hu CD45 | 100 Tests | Invitrogen |
| PE-Cy7 mouse IgG1 κ Iso Control | 20 Tests | Invitrogen |

**Table 3. Instruments and equipment**

| Name | Model No. | Manufacturer |
|---|---|---|
| CO₂ incubator | 3111 | Thermo |
| Microscope | IX53 | Olympus |
| Electronic balance | EJ-1201C | Beijing Langke Xingye Weighing Equipment Co., Ltd. |
| SF2000 three-button electronic digital display caliper | 111N-101B | Guilin Guanglu Measuring Instrument Co., Ltd. |
| ACEA NovoCyte flow cytometer | 3130 | ACEA |

### 4. Experimental animals and accommodation management

### 4.1 Experimental animals

Species: *Mus Musculus,* NCG (NOD-Prkdc^{em26Cd52}Il2rg^{em26Cd22}/Nju); female; weight: 18-22 g; quantity: 55; supplier: GemPharmatech.

### 4.2 Accommodation management

Accommodation management: the experimental animals were housed in individually ventilated cages in an SPF-grade clean laminar flow room of constant temperature and humidity, with 5 mice in each cage.

The temperature and humidity were controlled in ranges of 23 ± 3 °C and 40%-70%, respectively.

The cages were made of polycarbonate. The dimensions were 370 mm × 155 mm × 135 mm. The cages were paved with autoclaved and clean soft corn cob padding, which was refreshed twice a week. The cages were labeled with the number of animals, sex, species, time of receipt, grouping and start of treatment. Feed and water: SPF grade murine diet, disinfected by cobalt 60 radiation. The drinking water was ultrafiltered and autoclaved water. The animals had free access to sterile food and water.

The animals were numbered by ear tags.

### 5. Methodology

### 5.1 Cell culture

MDA-MB-436 tumor cells (YK-CL-075) were purchased from ATCC. The tumor cells were cultured in the Leibovitz's L-15 medium containing inactivated 10% fetal bovine serum, 100 U/mL of penicillin and 100 µg/mL of streptomycin in a 37 °C/5% CO₂ incubator, and were passaged every 3 to 4 days when the cells were overgrown. The tumor cells in logarithmic phase were used for *in vivo* tumor grafting.

PBMCs (human peripheral blood mononuclear cells) were purchased from ALLCELLS (Donor#: A0075).

### 5.2 Tumor cell grafting and grouping

MDA-MB-436 tumor cells were resuspended in a 1:1 (v:v) mixture of PBS and Matrigel at a concentration of 1×10⁸ cells/mL, and were grafted subcutaneously at the right flank of mice at 100 µL/mouse. When the tumor grew to about 1000 mm³, the tumors were aseptically removed and cut into small pieces having dimensions of about 2 mm × 2 mm × 2 mm, which were grafted subcutaneously at the right flank of experimental animals. After 2 weeks, PBMCs were resuspended in PBS and grafted into mice in an amount of 2 × 10⁶ cells/mouse. The animals were divided into 4 groups of 10 and treated (the day was recorded as PG-D0) when the tumor grew to about 93 mm³. The specific dosing regimen is shown in Table 4.

### 5.3 Measurement and experimental parameters of mouse body weight

The long and short diameters of the tumor were measured twice every week using a vernier caliper for calculating the tumor volume, and the calculation formula was: volume = 0.5 × long diameter × short diameter².

The T/C value was calculated from the tumor volume, where T is the mean relative tumor volume (RTV) of each treatment group and C is the mean RTV of the control group. RTV is the ratio of tumor volume after the treatment to that before the treatment. Tumor growth inhibition (TGI, %) = (1 - T/C) × 100%.

Three weeks after PBMC grafting, peripheral blood of the mice was collected, and the proportion of human-derived CD45⁺ cells (hCD45 cells) therein was determined by flow cytometry (FACS).

After the end of study, the tumors was removed, weighed and photographed.

### 6. Dosing regimen

**Table 4. Dosing regimen**

| Number of animals | Treatment | Dose (mg/kg)^{∗} | Route of administration | Frequency of administration^{#} |
|---|---|---|---|---|
| 10 | Vehicle (normal saline + PBS) | - | p.o. + i.p. | qod × 13 + q4d × 7 |
| 10 | Mefuparib hydrochloride | 40 | p.o. | qod × 13 |
| 10 | Tuoyi PD-1 monoclonal antibody injection | 10 | i.p. | q4d × 7 |
| 10 | Mefuparib hydrochloride | 40 | p.o. | qod × 13 |
| | Tuoyi PD-1 monoclonal antibody injection | 10 | i.p. | q4d × 7 |

| | | | | |
|---|---|---|---|---|
| Note: "^{∗}" the administration volume was determined as per 10 µL/g body weight, and the treatment can be discontinued until the body weight returned to normal level if body weight was reduced by 15-20%; i.p.: intraperitoneal injection; p.o.: per os; qod × 13: once every other day for 13 doses; q4d ×7: once every four days for 7 doses. | | | | |

### 7. Statistical analysis

Inter-group statistical analysis was performed on tumor volume using IBM SPSS Statistics 22.0 software and mixed linear model analysis. Inter-group statistical analysis was performed on tumor weight using One-Way ANOVA analysis, with p < 0.05 indicating significant differences.

### Example 1. Test results

### 1. Reaction and change in body weight of experimental animals after treatment

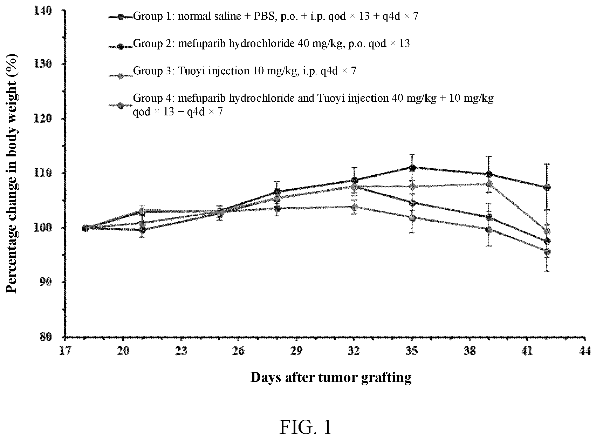

During the treatment, no significant acute adverse events were observed after treatment in all groups. In the middle and later stages of the study, mice in all groups showed weight loss, as shown in FIG. 1. The study demonstrated the good safety of the combined use of the mefuparib hydrochloride and the Tuoyi PD-1 monoclonal antibody injection.

### 2. Results of tumor growth inhibition

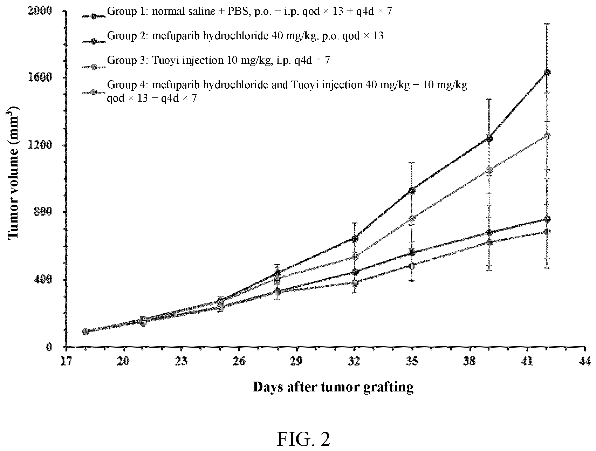

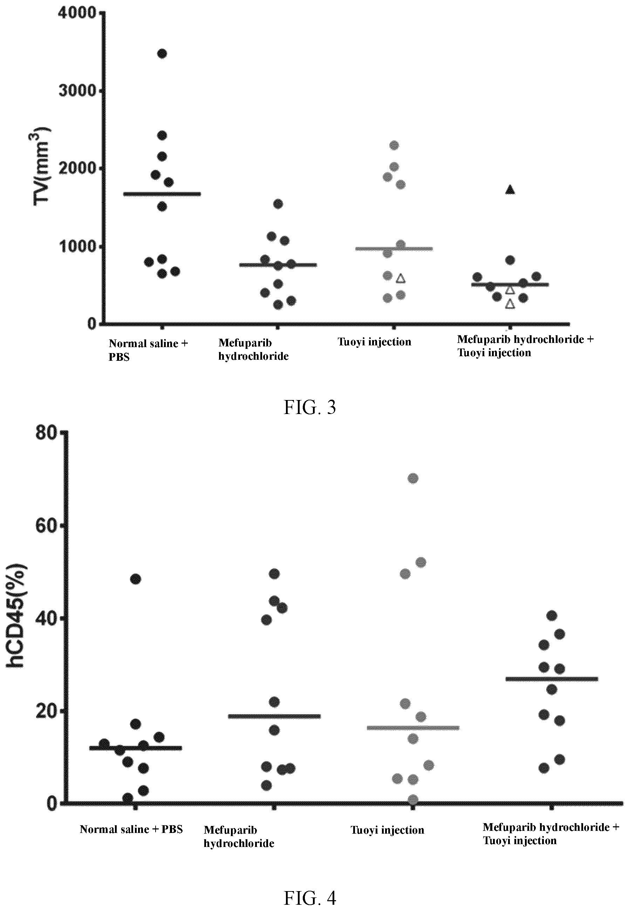

The change in tumor volume of the treated experimental animals is shown in Table 5, FIG. 2 and FIG. 3.

**Table 5. Tumor (tumor volume) inhibition of test compounds on MDA-MB-436 model**

| Group | Number of animals (mouse) | Tumor volume (mm³)^{a} | | Tumor growth inhibition (%) | P |
|---|---|---|---|---|---|
| | | Grouping (PG-D0) | Administration (PG-D24) | | |
| Vehicle (normal saline + PBS) | 10 | 93 ± 7 | 1632 ± 292 | -- | -- |
| Tuoyi PD-1 monoclonal antibody injection | 10 | 94 ± 7 | 1256 ± 252 | 29% | 0.010 |
| Mefuparib hydrochloride | 10 | 93 ± 6 | 761 ± 130 | 54% | 0.000 |
| Mefuparib hydrochloride + Tuoyi PD-1 monoclonal antibody injection | 10 | 93 ± 6 | 687 ± 160 | 60% | 0.000 |

Conclusion: on day 24 after grouping (PG-D24), the tumor growth inhibitions were 54%, 29% and 60% for the mefuparib hydrochloride group, the Tuoyi PD-1 monoclonal antibody injection group and the mefuparib hydrochloride + Tuoyi PD-1 monoclonal antibody injection group, respectively. The tumor volumes in all treatment groups were significantly smaller than that of the control group (p < 0.05); the tumor volume in the mefuparib hydrochloride + Tuoyi PD-1 monoclonal antibody injection group was significantly smaller than that of the Tuoyi PD-1 monoclonal antibody injection group (p < 0.05). These indicated that the combination produced efficacy significantly better than that of PD-1 monotherapy and superior therapeutic trend to that of mefuparib hydrochloride.

### 3. FACS assay on peripheral blood

FACS assay results of hCD45 in peripheral blood of mice 3 weeks after PBMC grafting are shown in FIG. 4.

As can be seen from FIG. 4, FACS assay results of hCD45 cells in peripheral blood of the mice were positive, indicating that the humanized immune reconstruction modeling succeeded, and the percentage medians of hCD45 cells in the mefuparib hydrochloride group, the Tuoyi PD-1 monoclonal antibody injection group, and the mefuparib hydrochloride group + the Tuoyi PD-1 monoclonal antibody injection group were all higher than that in the control group, wherein the percentage median of the mefuparib hydrochloride group + the Tuoyi PD-1 monoclonal antibody injection group was higher than those in the mefuparib hydrochloride group and the Tuoyi PD-1 monoclonal antibody injection group.

In conclusion, in the PBMC humanized MDA-MB-436 subcutaneous tumor grafting model, the combination of the Tuoyi PD-1 monoclonal antibody injection and the mefuparib hydrochloride generates a statistically significant anti-tumor effect, and can effectively inhibit the tumor growth. It demonstrates a tumor growth inhibition significantly higher than that of the Tuoyi PD-1 monoclonal antibody injection and an increased proportion of hCD45 cells in peripheral blood of mice.

Although specific embodiments of the present invention have been described above, it will be appreciated by those skilled in the art that these embodiments are merely illustrative and that many changes or modifications can be made to these embodiments without departing from the principles and spirit of the present invention. The scope of protection of the present invention is therefore defined by the appended claims.

## Claims

1. A pharmaceutical combination, comprising: a compound of formula A, a pharmaceutically acceptable salt thereof, a solvate thereof or a solvate of the pharmaceutically acceptable salt thereof; and "a PD-1 inhibitor and/or a PD-L1 inhibitor";

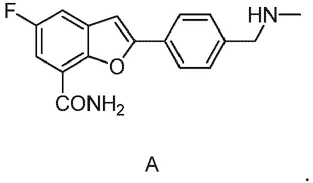

2. The pharmaceutical combination according to claim 1, wherein the pharmaceutically acceptable salt of the compound of formula A is mefuparib hydrochloride;
and/or the PD-1 inhibitor is one or more of a PD-1 antibody, a PD-1 polypeptide inhibitor and a PD-1 micromolecular inhibitor, preferably one or more of toripalimab, sintilimab, camrelizumab, pembrolizumab and nivolumab, more preferably toripalimab, and even more preferably Tuoyi;
and/or the PD-L1 inhibitor is one or more of a PD-L1 antibody, a PD-L1 polypeptide inhibitor and a PD-L1 micromolecular inhibitor, and preferably one or more of atezolizumab, durvalumab and alirocumab;
and/or "the PD-1 inhibitor and/or the PD-L1 inhibitor" and the "compound of formula A, the pharmaceutically acceptable salt thereof, the solvate thereof or the solvate of the pharmaceutically acceptable salt thereof" are administered simultaneously or separately, preferably sequentially;
and/or "the PD-1 inhibitor and/or the PD-L1 inhibitor" is administered orally or by injection;
and/or the compound of formula A, the pharmaceutically acceptable salt thereof, the solvate thereof or the solvate of the pharmaceutically acceptable salt thereof is administered orally.

3. The pharmaceutical combination according to claim 1, wherein the pharmaceutical combination comprises mefuparib hydrochloride and toripalimab.

4. A pharmaceutical composition X, comprising:
the compound of formula A, the pharmaceutically acceptable salt thereof, the solvate thereof or the solvate of the pharmaceutically acceptable salt thereof described in any one of claims 1 to 3;
"the PD-1 inhibitor and/or the PD-L1 inhibitor" described in any one of claims 1 to 3;
and a pharmaceutically acceptable excipient.

5. The pharmaceutical composition X according to claim 4, wherein the pharmaceutical composition X is in an injectable dosage form or an oral dosage form.

6. A pharmaceutical composition Y, comprising: a first pharmaceutical composition, and a second pharmaceutical composition; wherein
the first pharmaceutical composition comprises: the compound of formula A, the pharmaceutically acceptable salt thereof, the solvate thereof or the solvate of the pharmaceutically acceptable salt thereof described in any one of claims 1 to 3, and a pharmaceutically acceptable excipient;
the second pharmaceutical composition comprises: "the PD-1 inhibitor and/or the PD-L1 inhibitor" described in any one of claims 1 to 3, and a pharmaceutically acceptable excipient.

7. The pharmaceutical composition Y according to claim 6, wherein the first pharmaceutical composition is in an oral dosage form, preferably in a tablet dosage form;
and/or, the second pharmaceutical composition is in an injectable dosage form.

8. A kit, comprising:
a first container comprising the first pharmaceutical composition described in claim 6 or 7; and
a second container comprising the second pharmaceutical composition described in claim 6 or 7.

9. Use of a substance M in preparing a medicament for preventing and/or treating a tumor, wherein the substance M is the pharmaceutical combination described in any one of claims 1 to 3, the pharmaceutical composition X described in claim 4 or 5, or the pharmaceutical composition Y described in claim 6 or 7.

10. The use according to claim 9, wherein the tumor is a solid and/or hematological tumor, and in particular one or more of lung cancer, colon cancer, rectal cancer, breast cancer, prostate cancer, liver cancer, pancreatic cancer, brain cancer, kidney cancer, ovarian cancer, stomach cancer, skin cancer, bone cancer, neuroglioma, glioblastoma, hepatocellular carcinoma, papillary kidney cancer, head and neck cancer, leukemia, lymphoma, myeloma and multiple myeloma; and/or "the compound of formula A, the pharmaceutically acceptable salt thereof, the solvate thereof or the solvate of the pharmaceutically acceptable salt thereof" and "the PD-1 inhibitor and/or the PD-L1 inhibitor" are administered simultaneously or separately, preferably sequentially;
and/or the compound of formula A, the pharmaceutically acceptable salt thereof, the solvate thereof or the solvate of the pharmaceutically acceptable salt thereof is administered at a dose of 100-1000 mg;
and/or the compound of formula A, the pharmaceutically acceptable salt thereof, the solvate thereof or the solvate of the pharmaceutically acceptable salt thereof is administered at a frequency of 0.5-2 doses/day;
and/or the compound of formula A, the pharmaceutically acceptable salt thereof, the solvate thereof or the solvate of the pharmaceutically acceptable salt thereof is administered orally;
and/or "the PD-1 inhibitor and/or the PD-L1 inhibitor" is administered at a dose of 50-500 mg;
and/or "the PD-1 inhibitor and/or the PD-L1 inhibitor" is administered at a frequency of once every 7-31 days;
and/or "the PD-1 inhibitor and/or the PD-L1 inhibitor" is administered orally or by injection.

11. The use according to claim 10, wherein the compound of formula A, the pharmaceutically acceptable salt thereof, the solvate thereof or the solvate of the pharmaceutically acceptable salt thereof is administered orally at a dose of 100-1000 mg and at a frequency of 0.5-2 doses/day;
and "the PD-1 inhibitor and/or the PD-L1 inhibitor" is administered by injection at a dose of 50-500 mg and at a frequency of once every 7-31 days.

12. Use of "the compound of formula A, the pharmaceutically acceptable salt thereof, the solvate thereof or the solvate of the pharmaceutically acceptable salt thereof" described in any one of claims 1 to 3 in preparing a medicament for treating a tumor, wherein the compound of formula A or the pharmaceutically acceptable salt thereof is used in combination with "the PD-1 inhibitor and/or the PD-L1 inhibitor" described in any one of claims 1 to 3;
preferably, the tumor is a solid and/or hematological tumor, or in particular one or more of lung cancer, colon cancer, rectal cancer, breast cancer, prostate cancer, liver cancer, pancreatic cancer, brain cancer, kidney cancer, ovarian cancer, stomach cancer, skin cancer, bone cancer, neuroglioma, glioblastoma, hepatocellular carcinoma, papillary kidney cancer, head and neck cancer, leukemia, lymphoma, myeloma and multiple myeloma.

13. Use of "the PD-1 inhibitor and/or the PD-L1 inhibitor" described in any one of claims 1 to 3 in preparing a medicament for treating a tumor, wherein "the PD-1 inhibitor and/or the PD-L1 inhibitor" is used in combination with "the compound of formula A, the pharmaceutically acceptable salt thereof, the solvate thereof or the solvate of the pharmaceutically acceptable salt thereof" described in any one of claims 1 to 3;
preferably, the tumor is a solid and/or hematological tumor, and the solid tumor is one or more of lung cancer, colon cancer, rectal cancer, breast cancer, prostate cancer, liver cancer, pancreatic cancer, brain cancer, kidney cancer, ovarian cancer, stomach cancer, skin cancer, bone cancer, neuroglioma, glioblastoma, hepatocellular carcinoma, papillary kidney cancer, head and neck cancer, leukemia, lymphoma, myeloma and multiple myeloma.

14. A method for treating or preventing a tumor, comprising administering to a subject an effective amount of a substance M, wherein the substance M is the pharmaceutical combination described in any one of claims 1 to 3, the pharmaceutical composition X described in claim 4 or 5, or the pharmaceutical composition Y described in claim 6 or 7;
preferably, "the compound of formula A, the pharmaceutically acceptable salt thereof, the solvate thereof or the solvate of the pharmaceutically acceptable salt thereof" and "the PD-1 inhibitor and/or the PD-L1 inhibitor" are administered through the same route of administration and at the same dose and frequency as those described in claim 10 or 11.
